(19) Europäisches Patentamt
European Patent Office
Office européen des brevets

(11) **EP 3 514 235 B1**

(12) **EUROPÄISCHE PATENTSCHRIFT**

(45) Veröffentlichungstag und Bekanntmachung des Hinweises auf die Patenterteilung:
**03.01.2024 Patentblatt 2024/01**

(21) Anmeldenummer: **18152422.4**

(22) Anmeldetag: **18.01.2018**

(51) Internationale Patentklassifikation (IPC):
**C12N 15/113** (2010.01)   **A61K 31/7088** (2006.01)
**A61P 1/00** (2006.01)

(52) Gemeinsame Patentklassifikation (CPC):
**C12N 15/113; C12N 15/1136;** C12N 2310/127

(54) **ZUSAMMENSETZUNG ZUR BEHANDLUNG EINES AN COLITIS ULCEROSA LEIDENDEN PATIENTEN SOWIE VERWENDUNG DER ZUSAMMENSETZUNG ALS ARZNEIMITTEL**

COMPOSITION FOR THE TREATMENT OF A PATIENT SUFFERING FROM ULCERATIVE COLITIS AND UTILISATION OF SAID COMPOSITION AS MEDICAMENT

COMPOSITION DESTINÉE AU TRAITEMENT DE PATIENTS SOUFFRANT D'UNE COLITE ULCEREUSE AINSI QUE L'UTILISATION DE LADITE COMPOSITION EN TANT QUE MÉDICAMENT

(84) Benannte Vertragsstaaten:
AL AT BE BG CH CY CZ DE DK EE ES FI FR GB GR HR HU IE IS IT LI LT LU LV MC MK MT NL NO PL PT RO RS SE SI SK SM TR

(43) Veröffentlichungstag der Anmeldung:
**24.07.2019 Patentblatt 2019/30**

(73) Patentinhaber: **Sterna Biologicals GmbH**
**35037 Marburg (DE)**

(72) Erfinder: **Renz, Jonas**
**81675 München (DE)**

(74) Vertreter: **Pharma Patents International AG**
**Leonhardsgraben 52**
**Postfach**
**4001 Basel (CH)**

(56) Entgegenhaltungen:
WO-A1-2017/205506    WO-A2-2005/033314
WO-A2-2014/040891    US-A1- 2003 040 497

- POPP VANESSA ET AL: "Rectal Delivery of a DNAzyme That Specifically Blocks the Transcription Factor GATA3 and Reduces Colitis in Mice", GASTROENTEROLOGY, W.B. SAUNDERS CO, US, Bd. 152, Nr. 1, 14. September 2016 (2016-09-14), Seiten 176-192, XP029851140, ISSN: 0016-5085, DOI: 10.1053/J.GASTRO.2016.09.005
- Anonymous: "DRKS-ID: DRKS00007193 SB012 for the Treatment of Active Ulcerative Colitis (SECURE): a Prospective, Single-centre, Randomised, Double-blind, Placebo-controlled Phase IIa Clinical Trial to Evaluate the Efficacy, Pharmacokinetics, Tolerability, and Safety of SB012 Enema Administered Once Daily", , 9. Juni 2015 (2015-06-09), XP055476967, Gefunden im Internet: URL:http://www.drks.de/drks_web/navigate.do?navigationId=trial.HTML&TRIAL_ID=DRKS000 07193 [gefunden am 2018-05-18]
- WEIGMANN BENNO ET AL: "Suppression of Experimental Ulcerative Colitis by Topical Delivery of Specific Dnazyme Blocking Thetranscription Factor GATA3", GASTROENTEROLOGY, W.B. SAUNDERS CO, US, 22. April 2017 (2017-04-22), XP085104521, ISSN: 0016-5085, DOI: 10.1016/S0016-5085(17)32631-8
- KATRINA RAY: "A role for GATA3 in ulcerative colitis", NATURE REVIEWS / GASTROENTEROLOGY & HEPATOLOGY, Bd. 13, Nr. 11, 5. Oktober 2016 (2016-10-05), Seiten 624-624, XP55476486, US ISSN: 1759-5045, DOI: 10.1038/nrgastro.2016.163
- ALESYA A. FOKINA ET AL: "Delivery of therapeutic RNA-cleaving oligodeoxyribonucleotides (deoxyribozymes): from cell culture studies to clinical trials", EXPERT OPINION ON DRUG DELIVERY, Bd. 14, Nr. 9, 7. Dezember 2016 (2016-12-07), Seiten 1077-1089, XP55476495, GB ISSN: 1742-5247, DOI: 10.1080/17425247.2017.1266326

- URBANSKA ALEKSANDRA M ET AL: "What's Next for Gastrointestinal Disorders: No Needles?", JOURNAL OF CONTROLLED RELEASE, Bd. 221, 2. Dezember 2015 (2015-12-02), Seiten 48-61, XP029383249, ISSN: 0168-3659, DOI: 10.1016/J.JCONREL.2015.11.031
- Zhijie Xu ET AL: "Small-molecule Nucleic-acid-based Gene-silencing Strategies" In: "Nucleic Acids - From Basic Aspects to Laboratory Tools", 16. März 2016 (2016-03-16), InTech, XP55476951, ISBN: 978-953-51-2264-7 DOI: 10.5772/62137, * Tabelle 2 *
- HOLGER GARN ET AL: "GATA-3-specific DNAzyme - A novel approach for stratified asthma therapy", EUROPEAN JOURNAL OF IMMUNOLOGY, Bd. 47, Nr. 1, 2. Dezember 2016 (2016-12-02), Seiten 22-30, XP055476945, ISSN: 0014-2980, DOI: 10.1002/eji.201646450
- COMINELLI FABIO ET AL: "Uncovering Pathogenic Mechanisms of Inflammatory Bowel Disease Using Mouse Models of Crohn's Disease-Like Ileitis: What is the Right Model?", CELLULAR AND MOLECULAR GASTROENTEROLOGY AND HEPATOLOGY JUL 2017, vol. 4, no. 1, July 2017 (2017-07), pages 19-32, ISSN: 2352-345X
- KOELINK PJ AND TE VELDE AA: "Mistakes in mouse models of IBD and how to avoid them.", UEG EDUCATION, vol. 16, 2016, pages 11-14,
- NGUYEN THI LOAN ANH ET AL: "How informative is the mouse for human gut microbiota research?", DISEASE MODELS & MECHANISMS JAN 2015, vol. 8, no. 1, January 2015 (2015-01), pages 1-16, ISSN: 1754-8411
- ALDARS-GARCÍA LAILA ET AL: "The Interplay between Immune System and Microbiota in Inflammatory Bowel Disease: A Narrative Review.", INTERNATIONAL JOURNAL OF MOLECULAR SCIENCES 17 MAR 2021, vol. 22, no. 6, 17 March 2021 (2021-03-17) , ISSN: 1422-0067
- CROUWEL FEMKE ET AL: "Gut microbiota-driven drug metabolism in inflammatory bowel disease.", JOURNAL OF CROHN'S & COLITIS 11 JUL 2020, 11 July 2020 (2020-07-11), ISSN: 1876-4479
- VALATAS VASSILIS ET AL: "The value of experimental models of colitis in predicting efficacy of biological therapies for inflammatory bowel diseases.", AMERICAN JOURNAL OF PHYSIOLOGY. GASTROINTESTINAL AND LIVER PHYSIOLOGY DEC 2013, vol. 305, no. 11, December 2013 (2013-12), pages G763-G785, ISSN: 1522-1547
- VICTOR JULIAN ET AL: "Inability of DNAzymes to cleave RNA in vivo is due to limited Mg[Formula: see text] concentration in cells.", EUROPEAN BIOPHYSICS JOURNAL : EBJ MAY 2018, vol. 47, no. 4, May 2018 (2018-05), pages 333-343, ISSN: 1432-1017
- TRAPANI VALENTINA ET AL: "Dietary Magnesium Alleviates Experimental Murine Colitis Through Upregulation of the Transient Receptor Potential Melastatin 6 Channel.", INFLAMMATORY BOWEL DISEASES 15 09 2018, vol. 24, no. 10, 15 September 2018 (2018-09-15), pages 2198-2210, ISSN: 1536-4844
- SWANSON KENNETH D ET AL: "Reproducing the human mucosal environment ex vivo: inflammatory bowel disease as a paradigm.", CURRENT OPINION IN GASTROENTEROLOGY 11 2018, vol. 34, no. 6, November 2018 (2018-11), pages 384-391, ISSN: 1531-7056

Bemerkungen:

Das gesamte Dokument mit Referenztabelle(n) und Sequenzliste(n) kann von der Webseite des EPA heruntergeladen werden

**Beschreibung**

Die Erfindung betrifft eine Zusammensetzung zur Behandlung eines an einer mit chronischen Entzündungen einhergehenden Darmerkrankung leidenden Patienten nach Anspruch 1.

Falls nicht anders angegeben, werden die Begriffe "Expression" und "Genexpression" im Folgenden synonym verwendet.

Chronische Entzündungen sind in der Medizin von großer Bedeutung. Entsprechend wichtig ist es, Therapien für Erkrankungen wie zum Beispiel Darmerkrankungen zu finden, die mit chronischen Entzündungen einhergehen. Ein bekanntes Beispiel für eine solche Darmerkrankung ist Colitis ulcerosa.

Neben Morbus Crohn ist Colitis ulcerosa eine der weltweit am häufigsten auftretenden idiopathischen und entzündungsabhängigen Darmkrankheiten. Sie ist durch einen entzündlichen Befall des Dickdarms bzw. des Colons gekennzeichnet, der sich - anders als etwa bei Morbus Crohn - nur auf den Dickdarm und dort nur auf die Darmschleimhaut beschränkt.

An der Entstehung von chronischen Entzündungsreaktionen sowie von (oftmals auch entzündungsabhängigen) Autoimmunerkrankungen sind unter anderem zwei Transkriptionsfaktoren beteiligt: der Th1-zellspezifische Transkriptionsfaktor Tbet und der Th2-zellspezifische Transkriptionsfaktor GATA-3. Dabei induziert Tbet die spezifische Entwicklung von Th1-Zellen; GATA-3 induziert hingegen die spezifische Entwicklung von Th2-Zellen. Die Balance zwischen Th1- und Th2-Zellen wird also durch die Expression von GATA-3 bzw. durch die Inhibition von Tbet zugunsten der Th2-Zellen verschoben. Analog sorgen die Inhibition von GATA-3 bzw. die Expression von Tbet für ein steigendesTh1-Zellen-Niveau. Der Colitis ulcerosa liegen zum Beispiel entzündete Bereiche der Dickdarmschleimhaut zugrunde, deren GATA-3-mRNA-Niveaus signifikant erhöht sind (= erhöhte GATA-3-Expression), die also Th2-abhängig sind.

Zu dem speziellen Gleichgewicht zwischen GATA-3 und Tbet bzw. zwischen Th1- und Th2-Zellen sind zahlreiche wissenschaftliche Publikationen erschienen. Folglich sind in den letzten Jahren Versuche unternommen worden, die Transkriptionsfaktoren GATA-3 und Tbet spezifisch zu hemmen bzw. "auszuschalten". Eine Möglichkeit, das zu bewerkstelligen, stellt die Inhibition der Genexpression dieser beiden Transkriptionsfaktoren dar, wozu die moderne Gentechnologie diverse "Werkzeuge" zur Verfügung stellt. Eines dieser Werkzeuge umfasst DNAzyme.

DNAzyme repräsentieren eine verhältnismäßig neue Klasse von antisense-Molekülen. Als antisense-Moleküle werden Moleküle bezeichnet, die fähig sind, einzelsträngige Nukleinsäuren (i.d.R. mRNA) zu binden. Eine Besonderheit von DNAzymen ist dabei, dass sie neben dieser Bindefunktion auch eine katalytische Funktion aufweisen. Sie sind also in der Lage, einzelsträngige Ziel-DNA oder -RNA spezifisch zu spalten und sie somit abzubauen (Sel et al. 2008). Man spricht in diesem Fall auch von posttranskriptioneller Inhibition, weil die Inhibition der Genexpression auf mRNA Ebene stattfindet, also noch bevor sich die Translation der mRNA in ein Protein anschließen kann. Üblicherweise werden dazu DNAzyme des 10-23-Modells verwendet (Sontoro et al., 1997). Derartige DNAzyme besitzen eine katalytische Domäne von 15 Nukleotiden, welche von zwei Substratbindungsdomänen flankiert wird. Die katalytische Domäne kann dabei insbesondere die konservierte Sequenz *ggctagctacaacga* umfassen. Die angegebene Sequenz *ggctagctacaacga* ist lediglich eine bevorzugte Ausführungsform. Dem Fachmann ist bekannt, dass DNAzyme des 10-23-Modells mit abgewandelter katalytischer Domäne eine vergleichbare biologische Aktivität besitzen können. Die Länge der Substratbindungsdomänen ist hingegen variabel, wobei sich auch zwei korrespondierende Substratbindungsdomänen voneinander unterscheiden können. In einer bevorzugten Ausführung, beträgt die Länge der Substratbindungsdomänen zwischen 6 und 14 Nukleotide, wobei eine Länge von 9 Nukleotiden besonders bevorzugt wird. Solche DNAzyme weisen eine spezifische Sequenz wie zum Beispiel *nnnnnnnnnggctagctacaacgannnnnnnnn* auf. In der Regel sind die Substratbindungsdomänen vollständig komplementär zu den Regionen, die die vorgesehene Spaltstelle der Ziel-mRNA flankieren - um die Ziel-RNA zu binden und zu spalten, muss das DNAzym jedoch nicht unbedingt vollständig komplementär sein. DNAzyme des 10-23 Typs spalten die Ziel-mRNA an Purin-Pyrimidin Abfolge-Sequenzen. DNAzyme werden ausschließlich chemisch hergestellt und gelten daher nicht als biologische Wirkstoffe.

DNAzyme sind ebenso bekannt wie ihre Verwendung zur spezifischen Inhibition der Expression von GATA-3 bzw. Tbet. So wird zum Beispiel in der WO 2005/033314 A2 die Wirkweise verschiedener DNAzyme beschrieben. Der Offenbarungsgehalt der WO 2005/033314 wird als technologischer Hintergrund der vorliegenden Erfindung angesehen. POPP VANESSA et al. ("Rectal Delivery of a DNAzyme That Specifically Blocks the Transcription Factor GATA3 and Reduces Colitis in Mice", GASTROENTEROLOGY, Bd. 152, Nr. 1, 14. September 2016, Seiten 176-192) beschreibt die Behandlung einer experimentellen, mit chronischen Entzündungen einhergehenden Darmerkrankung in Mäusen mit einem DNAzym, das GATA-3 inhibiert.

Probleme, die bei der Verwendung von DNAzymen als Wirkstoff auftreten können, sind oftmals auf die verhältnismäßig hohe Instabilität und die Sensibilität von Nukleinsäuren zurückzuführen. Sofern sie nicht in physiologisch günstigen Lösungen vorliegen, neigen Nukleinsäuren zur schnellen Degradierung, etwa durch enzymatischen Abbau oder physikalischen Stress - dies gilt insbesondere für einzelsträngige Nukleinsäuren, zu denen auch DNAzyme zählen. Des Weiteren können sich DNAzyme in ihrer Spezifität und somit in ihrer Effektivität als Wirkstoff unterscheiden. Darüber hinaus kann die Stabilität von DNAzymen insbesondere durch Änderungen des pH-Wertes der DNAzymhaltigen Lösungen beeinträchtigt werden. So kann zum Beispiel ein niedriger pH-Wert (saures Milieu) eine Hydrolyse der DNAzym-Moleküle zur Folge haben. Auch ein zu hoher pH-Wert (basisches Milieu) kann die Stabilität und somit die Funktionalität der DNAzyme beeinflussen, etwa indem Sekundärstrukturen der Moleküle verändert werden.

Bisher gängige Behandlungsstrategien von Patienten mit Darmerkrankungen wie zum Beispiel Colitis ulcerosa werden durch Aktivität, Auftreten (Proktitis, linksseitige Colitis und ausgedehnte Colitis) und Muster der Erkrankung (Krankheitsverlauf, Antwort auf vorangegangene Medikation, mögliche Nebenwirkungen der Therapien etc.) bestimmt. Weitere Faktoren, welche die Frage nach der therapeutischen Entscheidung maßgeblich beeinflussen, sind Krankheitsdauer, Alter bei Beginn und Begleiterkrankungen des Patienten. Die Aktivität der Erkrankung ist außerdem ein wichtiger Indikator für die Notwendigkeit der Einweisung des Patienten in ein Krankenhaus.

In der Regel werden an Colitis ulcerosa leidende Patienten zunächst mit Mesalazin (5-Aminosalicylsäure) behandelt, welche rektal und oral verabreichbar ist. Falls die Symptome der Krankheit danach bestehen bleiben, werden häufig Corticosteroide verabreicht, wobei Patienten mit schwerer Colitis ulcerosa zur Intensivierung der Corticosteroid-Therapie oftmals stationär behandelt werden müssen. Wenn auch die Corticosteroide nicht anschlagen und keine Verbesserung des Zustands des Patienten zur Folge haben, so kann als derzeitige *ultima ratio* eine Calcineurin-hemmende Therapie erfolgen, etwa mit Ciclosporin, Tacrolimus oder mit monoklonalen Antikörpern, die beispielsweise gegen TNF gerichtet sind.

Dabei eignet sich keiner der vorbenannten Therapiewege dazu, gezielt Darmerkrankungen zu behandeln, die mit Entzündungen einhergehen. Vielmehr sind die eingesetzten Substanzen für ihre stark immunsuppressive Wirkung bekannt. Darüber hinaus stehen die Stoffe mit häufig auftretenden Nebenwirkungen in Verbindung, wie zum Beispiel erhöhte Immunsuppression, Hepatotoxizität, Myokardhypertrophie gastrointestinale Beschwerden, Müdigkeit und Schwindel, Diarrhö, Erbrechen, Nephrotoxizität, Neurotoxizität, Tremor, Hypertonie, Insomnie, Depressionen, Krämpfe, Maskierung von Infektionen, Hyperkaliämie, Hyperglykämie, erhöhtes Tumorrisiko - um nur einige zu nennen.

Aufgabe der Erfindung ist es also, die Nachteile im Stand der Technik zu beseitigen und eine Zusammensetzung bereitzustellen, die zur Behandlung von Patienten geeignet ist, die an einer mit chronischen Entzündungen einhergehenden Darmerkrankung leiden. Insbesondere soll dabei ein Wirkstoff zur Verfügung gestellt werden, der zur effektiven Behandlung von Colitis ulcerosa geeignet ist, ohne dabei zu erheblichen Nebenbeschwerden zu führen. Ferner soll eine verbesserte Remission gewährleistet werden.

Die Aufgabe wird erfindungsgemäß durch eine Zusammensetzung zur Behandlung eines Menschen gelöst, der an einer mit chronischen Entzündungen einhergehenden Darmerkrankung leidet, wobei die Zusammensetzung mindestens ein DNAzym umfasst, das die Expression von GATA-3 spezifisch inhibiert, dadurch gekennzeichnet, dass die Konzentration des DNAzyms in der Zusammensetzung zwischen 0,75 mg/ml und 75 mg/ml ist; wobei das DNAzym die Sequenz hgd40 (GTGGATGGAggctagctacaacgaGTCTTGGA) aufweist; wobei die Zusammensetzung derart verabreicht wird, dass die verabreichte Dosis des DNAzyms bei 250 bis 500 mg pro Mensch pro Tag liegt. Das DNAzym bindet und spaltet *in vivo* die mRNA des Transkriptionsfaktors GATA-3, das als Protein eine zentrale Rolle bei der Entstehung von Th2-abhängigen chronischen Entzündungserkrankungen darstellt. Durch diese Spaltung kann die mRNA nicht mehr in ein funktionales Protein translatiert werden. Folglich wird das GATA-3 Proteinniveau signifikant minimiert.

Dem Fachmann ist bekannt, dass unter einer Herunterregulation einer Expression sowohl eine teilweise als auch eine vollständige Inhibition der Expression verstanden werden kann. In jedem Fall wird die Expression im Vergleich zum natürlichen Expressionsniveau signifikant verringert.

Dabei ist vorgesehen, dass das DNAzym die Sequenz hgd40 (GTGGATGGAggctagctacaacgaGTCTTGGA) aufweist. Diese Sequenz zeigt eine besonders hohe Enzymaktivität und spaltet GATA-3-mRNA mit einer sehr hohen Spezifität sowie mit hoher Effizienz. Folglich eignet sich ein DNAzym mit der Sequenz hgd40 hervorragend zur gezielten und effektiven Behandlung von Darmerkrankungen, die Th2-abhängig sind, die also mit einem erhöhten GATA-3-Spiegel korrespondieren. Denkbar sind aber auch DNAzyme mit anderen Sequenzen, mit denen die Expression von GATA-3 ebenfalls spezifisch inhibiert werden kann.

Nach einer vorteilhaften Ausgestaltung der Erfindung ist vorgesehen, dass die Konzentration des DNAzyms in der Zusammensetzung zwischen 0,75 mg/ml und 75 mg/ml ist. Dabei ist insbesondere vorgesehen, dass die Konzentration des DNAzyms in der Zusammensetzung ca. 7,5 mg/ml beträgt. Eine derartige Konzentration erweist sich als besonders effizient. DNAzym-Konzentrationen von unter 0,75 mg/ml können mit einem hohen Wirkungsverlust der Zusammensetzung in Verbindung stehen. Gleichzeitig kann sich bei Konzentrationen von über 75 mg/ml das Toxizitätsrisiko erhöhen, dass bei Verabreichung der Zusammensetzung bestehen kann. Hinsichtlich absoluter Mengen ist erfindungsgemäß vorgesehen, dass das DNAzym hgd40 in einer Dosis von 250 mg bis 500 mg pro Patient pro Tag verabreicht wird. Eine derartige Zusammensetzung hat überraschenderweise zur Folge, dass auch noch Wochen nach Darreichung der Zusammensetzung ein positiver Effekt des Wirkstoffs zu beobachten ist. Die Zusammensetzung eignet sich somit hervorragend zur Behandlung eines Patienten, der an einer mit chronischen Entzündungen einhergehenden Darmerkrankung leidet, weil auf diese Weise applizierte DNAzyme ihre Wirkung über einen langen Zeitraum hinweg entfalten. Die erfindungsgemäße Zusammensetzung führt also zu einem hohen Grad der Remission bei den mit der Zusammensetzung behandelten Patienten.

Es ist außerdem vorteilhaft, wenn die Zusammensetzung in Form einer wässrigen Lösung vorliegt. Eine solche Lösung ist günstig und einfach in der Herstellung. Außerdem hat eine wässrige Lösung keine erhöhte Toxizität, wodurch sie sich zur Verwendung in der Therapeutik eignet.

Ein weiterer Vorteil ergibt sich aus der Ausführungsvariante, nach der die Zusammensetzung mindestens ein Salz umfasst. Dabei ist insbesondere vorgesehen, dass das Salz Natriumchlorid und/oder Kaliumchlorid und/oder ein Phosphat ist. Erfindungsgemäß soll das hgd40 zur Verwendung in PBS gelöst vorliegen, weshalb die Zusammensetzung im Wesentlichen eine Phosphatgepufferte Salzlösung (PBS) ist, die das DNAzym als Wirkstoff enthält. Es sind jedoch auch andere in der Biochemisch gängige Puffer denkbar. Außerdem können weitere Additive wie zum Beispiel EDTA hinzugefügt werden.

Nach einer vorteilhaften Weiterentwicklung der Erfindung ist vorgesehen, dass die Zusammensetzung zur rektalen Darreichung geeignet ist. Hieraus ergibt sich der Vorteil, dass der DNAzym-Wirkstoff hgd40 am direkt Ort der Entzündung wirken kann, nämlich an der Oberflächenschleimhaut des Dickdarms. Dort wird Wirkstoff von der entzündeten Schleimhaut aufgenommen. Eine schnelle und effiziente Wirkung des Therapeutikums sind die Folge. Denkbar sind aber auch orale Verabreichungsformen wie zum Beispiel Kapseln, Pillen oder Tabletten.

Als weiteres Merkmal der Zusammensetzung kann vorgesehen sein, dass die Zusammensetzung bei einem gewünschten Therapiezeitraum von n Tagen während eines Verabreichungszeitraums von maximal n Tagen verabreicht wird. Dabei kann beabsichtig sein, dass der Verabreichungszeitraum bei einem gewünschten Therapiezeitraum von n Tagen maximal $\frac{3n}{4}$ Tage des gewünschten Therapiezeitraums umfasst. Insbesondere kann ein Verabreichungszeitraum von maximal $\frac{n}{2}$ Tagen des gewünschten Therapiezeitraums vorgesehen sein. Als Therapiezeitraum ist dabei der Zeitraum zu verstehen, während dessen ein Patient, der an einer mit chronischen Entzündungen einhergehenden Darmerkrankung leidet, behandelt werden soll (während dessen also der Wirkstoff der Zusammensetzung bei dem Patienten wirken und somit die Erkrankungssymptome bekämpfen soll).

Ein derartiges Dosierungsschema, bei dem die Zusammensetzung bei einem gewünschten Therapiezeitraum von n Tagen während eines Verabreichungszeitraums von maximal n Tagen verabreicht werden soll, ist nicht zuletzt deshalb möglich, weil die Zusammensetzung bei den behandelten Patienten zu einem hohen Grad der Remission führt. Das hat wiederum den vorteilhaften Effekt zur Folge, dass ein Patient nicht länger behandelt werden muss als nötig. Dies ist wünschenswert, weil es für eine Entlastung des zu behandelnden Patienten sorgt.

Ein weiterer Aspekt besteht in der Zusammensetzung zur Verwendung als Arzneimittel zur Behandlung eines an einer mit chronischen Entzündungen einhergehenden Darmerkrankung leidenden Patienten. Die Zusammensetzung kann auch als SB012 bezeichnet werden, sobald sie als Arzneimittel eingesetzt wird. Dabei kann die Erfindung insbesondere vorsehen, dass die Zusammensetzung in Form eines Zäpfchens appliziert wird. Alternativ kann vorgesehen sein, dass die Zusammensetzung in Form eines Einlaufs oder eines Rektalschaums appliziert wird. Auf vorteilhafte Weise kann die Zusammensetzung SB012 zur Behandlung betroffener Patienten also entweder als Arzneimittel im Rahmen einer unterstützenden Therapie oder im Zuge einer eigenen Therapie eingesetzt werden. Dabei ergibt sich insbesondere aus der Form der Zusammensetzung bzw. des Arzneimittels als Zäpfchen, Einlauf oder Rektalschaum eine einfache und schnelle Applizierbarkeit des Arzneimittels. Folglich kann das Arzneimittel ambulant und notfalls auch ohne Hilfe durch Fachpersonal verabreicht werden.

Die oben geschilderten Vorteile und Wirkungen ergeben sich insbesondere bei der Verwendung der erfindungsgemäßen Zusammensetzung bei der Behandlung eines Patienten, der an einer mit chronischen Entzündungen einhergehenden Darmerkrankung leidet.

Weitere Merkmale, Einzelheiten und Vorteile der Erfindung ergeben sich aus dem Wortlaut der Ansprüche sowie aus der folgenden Beschreibung von Ausführungsbeispielen und Figuren.

Es zeigen

Figur 1    Mittelwerte von Gesamt-MAYO-scores aus einer Versuchsreihe zur Untersuchung der erfindungsgemäßen Zusammensetzung in grafischer Darstellung;

Figur 2    Mittelwerte von Gesamt-MAYO-scores aus der Versuchsreihe aus Figur 1 in tabellarischer Darstellung;

Figur 3    Mittelwerte endoskopischer MAYO-scores des Sigmoids aus einer Versuchsreihe zur Untersuchung der erfindungsgemäßen Zusammensetzung als Balkendiagramm; und

Figur 4    Mittelwerte endoskopischer MAYO-scores aus der Versuchsreihe aus Figur 3 in tabellarischer Darstellung.

In einer Ausführungsform liegt die Zusammensetzung mit dem DNAzym hgd40 in einem pharmakologisch verträglichen Träger zusammen mit Hilfsstoffen und/oder Füllmitteln oder als Lösungsmittel vor.

Die Zusammensetzung kann dann zum Beispiel in Form von Zäpfchen (Suppositorien), Tropfen, Mundspray, Nasenspray, Pillen, Tabletten, Filmtabletten, Schichttabletten, Zäpfchen, Gelen, Salben, Sirup, Inhalationspulvern, Granulaten, Emulsionen, Dispersionen, Mikrokapseln, Kapseln, Puder oder Injektionslösungen hergestellt und verabreicht werden. Zudem umfasst die Gruppe der pharmakologisch verträglichen Träger Formulierungen wie Schichttabletten zur kontrollierten und/oder kontinuierlichen Freisetzung des Wirkstoffs sowie Mikroverkapselungen als spezielle Darreichungsform.

Derartige Formulierungen sind für die rektale Verabreichung gut geeignet. Es können aber auch andere Formen der Verabreichung vorgesehen sein, wie zum Beispiel Inhalation, intravenöse, intraperitoneale, intramuskuläre, subkutane, mukokutane, orale, transdermale, topikale, bukkale, intradermale, intragastrale, intrakutane, intranasale, intrabuccale, perkutane oder sublinguale Verabreichung.

Durch den Zusatz von Hilfsstoffen werden die pharmazeutischen Eigenschaften der Zusammensetzung für die spezielle Anwendung verbessert. Als pharmakologisch verträgliche Hilfsstoffe können beispielsweise Lactose, Stärke, Sorbitol, Sucrose, Cellulose, Magnesiumstearat, Dicalciumphosphat, Calciumsulfat, Talk, Mannitol, Ethylalkohol und dergleichen eingesetzt werden. Die vorbenannten Träger können bis zu 95% (w/w) aus einem derartigen Hilfsstoff bestehen. Für die Zubereitung von Suppositorien werden bevorzugt niedrigschmelzende Wachse, Fettsäureester und/oder Glyceride eingesetzt.

Bei der Verarbeitung geringer bis sehr geringer Mengen werden üblicherweise Füllmittel benötigt, welche dafür sorgen, dass die erfindungsgemäße Zusammensetzung die notwendige Größe/Masse erhält und problemlos verwendet werden kann. Als Füllstoff wird ein Stoff aus der Gruppe umfassend Stärken (Mais-, Kartoffel- und Weizenstärke), Lactose, Glucose, Mannitol, Sorbitol und Fructose verwendet.

Ferner können der Zusammensetzung noch Zerfallsmittel (Sprengmittel), Farbstoffe, Geschmacksstoffe und/oder Bindemittel zugesetzt werden. Als Zerfallsmittel werden Stoffe aus der Gruppe umfassend Stärken (Mais-, Kartoffel- und Weizenstärke, Natrium-Carboxymethylstärke), natürliche und synthetische Gummis wie beispielsweise Johannisbrotkernmehl, Karaya, Guar, Tragacanth, Agar, Cellulosederivate wie Methylcellulose, Natrium-Carboxymethylcellulose, microkristalline Cellulose, Alginate, Tonerden, Bentonite, PVP (Polyvinylpyrrolidon), Carbopol und Magnesiumperoxid eingesetzt. Diese Bestandteile können in Mengen von bis zu 30 % (w/w) eingesetzt werden. Zerfallsmittel (Sprengmittel) sorgen für eine gute Verpressbarkeit der Zusammensetzung durch Verbesserung der Partikelhaftung. Sie erleichtern außerdem ein späteres Zerfallen, etwa im Magen-Darm-Trakt. Sie wirken indem sie Feuchtigkeit absorbieren, die Kapillarität erhöhen und quellen oder unter Einfluss von Feuchtigkeit Gase entwickeln und aufbrausen oder indem sie die Benetzbarkeit der Tabletten als Hydrophilisierungsmittel erhöhen. Bindemittel sorgen für den Zusammenhalt in Granulaten und neben dem Pressdruck für die Festigkeit von Tabletten

Flüssige Formulierungen umfassen Lösungen, Suspensionen, Sprays und Emulsionen, wie zum Beispiel wässrige Injektionslösungen oder Lösungen auf Wasser-Propylenglycol-Basis für parenterale Injektionen.

Es können außerdem Gleitmittel vorgesehen. Gleitmittel sind ein Überbegriff für Fließmittel, Schmiermittel und Formentrennmittel. Fließmittel verbessern allgemein die Fließeigenschaften indem sie Reibungs- und Haftkräfte zwischen Schüttgutteilchen verringern. Sie verringern die interpartikulären Reibungen und reduzieren die Feuchtigkeit auf der Oberfläche.

Als Gleitmittel können Stoffe aus der Gruppe umfassend Borsäure, Stearate (z.B. Magnesiumstearat, Calciumstearat, Kaliumstearat), Stearinsäure, hochschmelzende Wachse und wasserlösliche Stoffe aus der Gruppe umfassend Natriumchlorid, Natriumbenzoat, Natriumacetat, Natriumoleat, Polyethylenglycol und Aminosäuren (z.B. Leucin) eingesetzt. Gleitmittel können in Mengen von bis zu 15 % (w/w) verwendet werden.

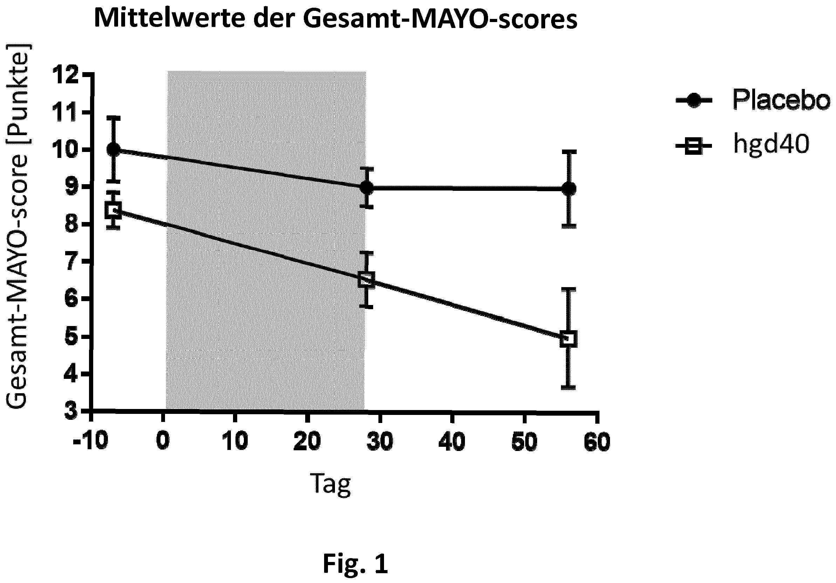

Den Figuren 1 bis 4 liegt jeweils dieselbe Versuchsreihe zu Grunde. Bei der Versuchsreihe wurden Patienten mit einem DNAzym behandelt, das die Sequenz *GTGGATGGAggctagctacaacgaGTCTTGGA* (hgd40) aufweist und das die Expression von GATA-3 spezifisch inhibiert bzw. herunterreguliert. Denkbar ist aber auch die Behandlung mit anderen DNAzymen, welche die Expression von GATA-3 spezifisch inhibieren oder herunterregulieren. Parallel zu der Behandlung mit dem DNAzym hgd40 wurde einer Kontrollgruppe eine PBS-Pufferlösung verabreicht. Diese Kontrollgruppe hat folglich kein DNAzym erhalten.

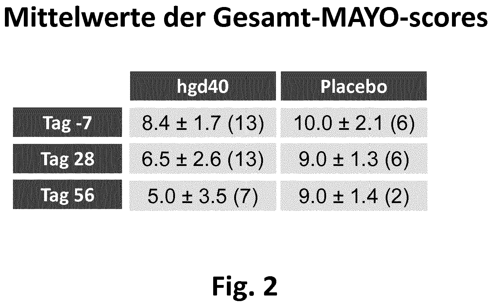

Fig. 1 und Fig. 2 stellen jeweils dieselben Ergebnisse dar. In Fig. 1 sind Mittelwerte von Gesamt-MAYO-scores aus der vorbenannten Versuchsreihe zur Untersuchung der erfindungsgemäßen Zusammensetzung in grafischer Darstellung gezeigt. In Fig. 2 sind Mittelwerte von Gesamt-MAYO-scores aus der Versuchsreihe aus Fig. 1 tabellarisch dargestellt. Der MAYO-score wird vor allem in klinischen Studien als Bestimmungsindex der Krankheitsaktivität von Colitis ulcerosa herangezogen. In diesen vierstufigen Index gehen die Stuhlfrequenz, die Stärke rektaler Blutungen, die endoskopische Beurteilung der Mukosa und die Gesamtbeurteilung durch einen Arzt ein. In dem Graph ist der Mittelwert der MAYO-scores von Patienten der Versuchsreihe (Y-Achse) gegen die Behandlungsdauer in Tagen (X-Achse) aufgetragen.

Bei der Versuchsreihe, die mit den in Fig. 1 und Fig. 2 gezeigten MAYO-scores korrespondiert, wurden Patienten mit einem DNAzym behandelt, das die Sequenz *GTGGATGGAggctagctacaacgaGTCTTGGA* aufweist und das die Expression von GATA-3 spezifisch inhibiert bzw. herunterreguliert (Fig.1 und 2, hgd40). Parallel zu der Behandlung mit dem DNAzym hgd40 wurde einer Kontrollgruppe eine PBS-Pufferlösung verabreicht (Fig.1 und 2, Placebo). Die Kontrollgruppe hat folglich kein DNAzym erhalten. Der Zeitraum, in dem die Patienten bzw. die Kontrollgruppe mit dem Wirkstoff bzw. mit dem Placebo behandelt worden sind, ist in Fig. 1 grau hinterlegt (28 Tage). Die MAYO-scores wurden dreimal gemessen: 7 Tage vor Beginn des Verabreichungszeitraums (-7), 28 Tage nach Beginn des Verabreichungszeitraums (28) und 28 Tage nach Ende des Verabreichungszeitraums (56).

In Fig. 1 sind die MAYO-scores der Tabelle aus Fig. 2 graphisch dargestellt. Dabei zeigt sich eindrucksvoll, dass der Mittelwert der MAYO-scores der Patienten, die mit hgd40 behandelt worden sind, in einem signifikant höheren Maße abnimmt, als der Mittelwert der MAYO-scores aus der Kontrollgruppe. Liegt der mittlere MAYO-score der behandelten Patienten zu Beginn der Versuchsreihe (-7) noch bei ca. 8,4, so liegt er bereits 28 Tage nach Beginn des Verabreichungszeitraums bei ca. 6,5. Am Ende der Versuchsreihe (56) liegt der mittlere MAYO-score der mit hgd40 behandelten Patienten bei ca. 5.0 (vgl. dazu Fig. 2). Insgesamt ist also eine Abnahme des mittleren MAYO-scores der Patienten von ca. 3,4 zu beobachten. Im Vergleich dazu verringert sich der mittlere MAYO-score der Kontrollgruppe nur um ca. 1,0 (von 10,0 auf 9,0; Fig. 2).

Die in Fig. 1 und Fig. 2 dargestellten Ergebnisse belegen eindrücklich, dass die erfindungsgemäße Zusammensetzung zur Behandlung eines Patienten geeignet ist, der an einer mit chronischen Entzündungen einhergehenden Darmerkrankung leidet.

Dabei ist von besonderem Vorteil, dass sich auch noch über einen Zeitraum von mindestens 28 Tagen nach Ende der letzten Verabreichung der Zusammensetzung ein positiver Effekt des Wirkstoffs beobachten lässt. Dieser Effekt zeigt sich darin, dass die MAYO-scores der behandelten Patienten auch noch nach Tag 28 (letzter Tag des Verabreichungszeitraums) über einen Zeitraum von mindestens vier weiteren Wochen abnehmen. Die erfindungsgemäße Zusammensetzung führt somit überraschenderweise bei den behandelten Patienten zu einem hohen Grad der Remission.

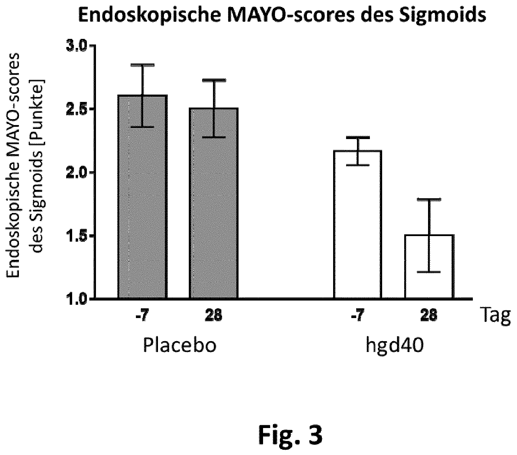

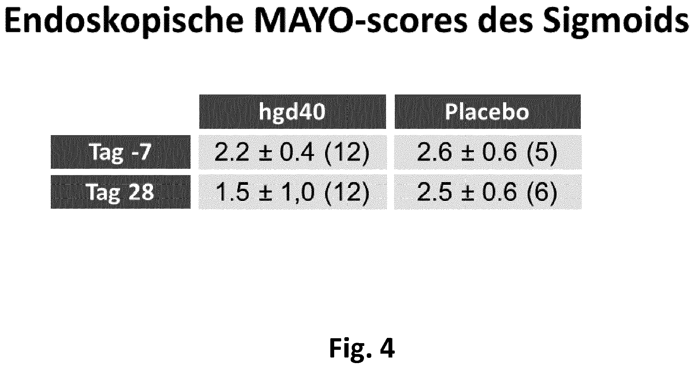

Fig. 3 und Fig. 4 liegt dieselbe Versuchsreihe zu Grunde, wie Fig. 1 und Fig. 2. Allerdings wurden die Ergebnisse, die mit Fig. 3 und Fig. 4 korrespondieren, unter anderen Gesichtspunkten erhalten. In Fig. 3 sind die Mittelwerte endoskopischer MAYO-scores des Sigmoids als Balkendiagramme dargestellt. Die zugehörigen Messdaten können Fig. 4 entnommen werden. Der Fachmann versteht unter dem Begriff "Sigmoid" den letzten Teil des menschlichen Dickdarms, Colon sigmoideum. Beim endoskopischen MAYO-score werden endoskopische Befunde hinsichtlich krankheitstypischer Charakteristika bewertet.

Analog zu Fig.1 und 2 wurden bei der Versuchsreihe, die mit den in Fig. 3 und Fig. 4 gezeigten endoskopischen MAYO-scores korrespondiert, Patienten mit einem DNAzym behandelt, das die Sequenz *GTGGATGGAggctagctacaacgaGTCTTGGA* aufweist und das die Expression von GATA-3 spezifisch inhibiert bzw. herunterreguliert (Fig. 3 und Fig. 4, "hgd40"). Denkbar ist aber auch im Falle von Fig. 3 und Fig. 4 die Behandlung mit anderen DNAzymen, welche die Expression von GATA-3 spezifisch inhibieren oder herunterregulieren. Parallel zu der Behandlung mit dem DNAzym hgd40 wurde einer Kontrollgruppe PBS-Pufferlösung verabreicht (Fig.3 und Fig. 4, Placebo). Diese Kontrollgruppe hat folglich kein DNAzym erhalten. Der Zeitraum, in dem die Patienten bzw. die Kontrollgruppe mit dem Wirkstoff bzw. mit dem Placebo behandelt worden sind, betrug 28 Tage. Die MAYO-scores wurden zweimal gemessen: 7 Tage vor Beginn des Verabreichungszeitraums (-7) und am 28. und letzten Tag des Verabreichungszeitraums (28).

Die Balkendiagramme der Fig. 3 geben einen Überblick über den Gesamtverlauf der Versuchsreihe. In dunkelgrau sind die Balken dargestellt, die mit den endoskopischen MAYO-scores der Kontrollgruppe korrespondieren (Fig.3, Placebo). Die weißen Balken in Fig. 3 korrespondieren mit den MAYO-scores der Patienten, die mit hgd40 behandelt worden sind (Fig. 3, hgd40). In Fig. 3 ist nun jeweils der Mittelwert der MAYO-scores zum ersten Zeitpunkt (7 Tage vor Beginn des Verabreichungszeitraums; -7) dem Mittelwert der MAYO-scores zum Ende des Verabreichungszeitraums (28. Tag des Verabreichungszeitraums; 28) gegenübergestellt. Dabei ist im Falle der Kontrollgruppe kein signifikanter Unterschied zu erkennen (vgl. dazu Fig. 3, graue Balken). Der Mittelwert der MAYO-scores betrug am Tag -7 ca. 2,6 und am Tag 28 ca. 2,5 (vgl. dazu Fig. 4, Placebo).

Demgegenüber stehen die Befunde der mit hgd40 behandelten Patienten. Bei den mit hgd40 behandelten Patienten wurden an ebenfalls zum ersten Zeitpunkt (-7) und zum Ende des Verabreichungszeitraums (28. Tag) MAYO-scores vergeben. Die Mittelwerte dieser MAYO-scores sind in Fig. 3 gegenübergestellt (Fig. 3, weiße Balken). Dabei ist bei der Gruppe der mit hgd40 behandelten Patienten eindeutig zu erkennen, dass zwischen dem Mittelwert von Tag -7 und dem Mittelwert von Tag 28 ein signifikanter Unterschied besteht. So verringert sich der mittlere MAYO-score von ca. 2,2 (Tag -7) auf ca. 1,5 (Fig. 4, hgd40).

Die in den Figuren 1 bis 4 dargestellten Ergebnisse belegen also eindrucksvoll, dass sich die erfindungsgemäße Zusammensetzung umfassend mindestens ein DNAzym, das die Expression von GATA-3 spezifisch inhibiert, zur Behandlung eines Patienten eignet, der an einer mit chronischen Entzündungen einhergehenden Darmerkrankung leidet. Insbesondere zeigen die Ergebnisse, dass die Beschwerden, die üblicherweise in Verbindung mit chronischen entzündeten Darmerkrankungen auftreten, wirksam vermindert werden können. Dabei zeigen vor allem die Ergebnisse der Figuren 1 und 2, dass die Zusammensetzung eine verbesserte Remission gewährleistet.

**Patentansprüche**

1. Zusammensetzung zur Verwendung in der Behandlung eines Menschen, der an einer mit chronischen Entzündungen einhergehenden Darmerkrankung leidet, wobei die Zusammensetzung mindestens ein DNAzym umfasst, das die Expression von GATA-3 spezifisch inhibiert, **dadurch gekennzeichnet, dass** die Konzentration des DNAzyms in der Zusammensetzung zwischen 0,75 mg/ml und 75 mg/ml ist, wobei das DNAzym hgd40 die Sequenz GTGGATGGAggctagctacaacgaGTCTTGGA aufweist; wobei die Zusammensetzung derart verabreicht wird, dass die verabreichte Dosis des DNAzyms bei 250 bis 500 mg pro Mensch pro Tag liegt.

2. Zusammensetzung zur Verwendung nach einem der vorhergehenden Ansprüche, **dadurch gekennzeichnet, dass** die Zusammensetzung in Form einer wässrigen Lösung vorliegt

3. Zusammensetzung zur Verwendung nach einem der vorhergehenden Ansprüche, **dadurch gekennzeichnet, dass** die Zusammensetzung mindestens ein Salz umfasst

4. Zusammensetzung zur Verwendung nach Anspruch 3, **dadurch gekennzeichnet, dass** das Salz Natriumchlorid und/oder Kaliumchlorid und/oder ein Phosphat ist.

5. Zusammensetzung zur Verwendung nach einem der vorhergehenden Ansprüche, **dadurch gekennzeichnet, dass** die Zusammensetzung zur rektalen Darreichung geeignet ist

6. Zusammensetzung zur Verwendung nach einem der vorhergehenden Ansprüche, **dadurch gekennzeichnet, dass** die Zusammensetzung bei einem gewünschten Therapiezeitraum von n Tagen während eines Verabreichungszeitraums von maximal n Tagen verabreicht wird, wobei insbesondere ein Verabreichungszeitraum von maximal $\frac{n}{2}$ Tagen vorgesehen ist.

7. Zusammensetzung zur Verwendung nach einem der Ansprüche 1 bis 6 , **dadurch gekennzeichnet, dass** die Zusammensetzung in Form eines Zäpfchens appliziert wird.

8. Zusammensetzung zur Verwendung nach einem der Ansprüche 1 bis 6 , **dadurch gekennzeichnet, dass** die Zusammensetzung in Form eines Einlaufs und/oder eines Rektalschaums appliziert wird.

**Claims**

1. Composition for use in treating a human suffering from a bowel disease associated with chronic inflammations, the composition comprising at least one DNAzyme which specifically inhibits the expression of GATA-3, **characterized in that** the concentration of the DNAzyme in the composition is between 0.75 mg/ml and 75 mg/ml; wherein the DNAzyme hgd40 has the sequence GTGGATGGAggctagctacaacgaGTCTTGGA; wherein the composition is administered such that the administered dose of the DNAzyme is from 250 to 500 mg per human per day.

2. Composition for use according to any of the preceding claims, **characterized in that** the composition is in the form of an aqueous solution.

3. Composition for use according to any of the preceding claims, **characterized in that** the composition comprises at least one salt.

4. Composition for use according to Claim 3, **characterized in that** the salt is sodium chloride and/or potassium chloride and/or a phosphate.

5. Composition for use according to any of the preceding claims, **characterized in that** the composition is suitable for rectal administration.

6. Composition for use according to any of the preceding claims, **characterized in that** the composition is, in the case of a desired therapy period of n days, administered over an administration period of not more than n days, especially an administration period of not more than n/2 days.

7. Composition for use according to any of Claims 1 to 6, **characterized in that** the composition is administered in the form of a suppository.

8. Composition for use according to any of Claims 1 to 6, **characterized in that** the composition is administered in the form of an enema and/or a rectal foam.

**Revendications**

1. Composition destinée à être utilisée dans le traitement d'une personne souffrant d'une maladie intestinale associée à des inflammations chroniques, la composition comprenant au moins une ADNzyme qui inhibe spécifiquement l'expression de GATA-3, **caractérisée en ce que** la concentration de l'ADNzyme dans la composition est comprise entre 0,75 mg/ml et 75 mg/ml ; l'ADNzyme hgd40 présentant la séquence GTGGATGGAggctagctacaacgaGTCTTGGA ; la composition étant administrée de manière à ce que la dose administrée de l'ADNzyme soit de 250 à 500 mg par personne et par jour

2. Composition destinée à être utilisée selon l'une des revendications précédentes, **caractérisée en ce que** la composition se présente sous forme d'une solution aqueuse.

3. Composition destinée à être utilisée selon l'une des revendications précédentes, **caractérisée en ce que** la composition comprend au moins un sel.

4. Composition destinée à être utilisée selon la revendication 3, **caractérisée en ce que** le sel est du chlorure de sodium et/ou du chlorure de potassium et/ou un phosphate.

5. Composition destinée à être utilisée selon l'une des revendications précédentes, **caractérisée en ce que** la composition est adaptée à une administration par voie rectale.

6. Composition destinée à être utilisée selon l'une des revendications précédentes, **caractérisée en ce que** la composition est administrée pendant une durée thérapeutique souhaitée de n jours pendant une durée d'administration d'un maximum de n jours, en particulier une durée d'administration d'un maximum de n/2 jours est prévue.

7. Composition destinée à être utilisée selon l'une des revendications 1 à 6, **caractérisée en ce que** la composition est appliquée sous forme de suppositoire.

8. Composition destinée à être utilisée selon l'une des revendications 1 à 6, **caractérisée en ce que** la composition est appliquée sous forme d'un lavement et/ou d'une mousse rectale.

**Mittelwerte der Gesamt-MAYO-scores**

Fig. 1

# Mittelwerte der Gesamt-MAYO-scores

| | hgd40 | Placebo |
|---|---|---|
| Tag -7 | 8.4 ± 1.7 (13) | 10.0 ± 2.1 (6) |
| Tag 28 | 6.5 ± 2.6 (13) | 9.0 ± 1.3 (6) |
| Tag 56 | 5.0 ± 3.5 (7) | 9.0 ± 1.4 (2) |

**Fig. 2**

Endoskopische MAYO-scores des Sigmoids

Fig. 3

Fig. 4

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2005033314 A2

- WO 2005033314 A

**In der Beschreibung aufgeführte Nicht-Patentliteratur**

- **POPP VANESSA et al.** Rectal Delivery of a DNAzyme That Specifically Blocks the Transcription Factor GATA3 and Reduces Colitis in Mice. *GASTROENTEROLOGY,* 14. September 2016, vol. 152 (1), 176-192